# FASCICULE DE BREVET EUROPEEN

(11) **EP 3 746 184 B1**
(45) Date de publication et mention de la délivrance du brevet: **19.01.2022**
(21) Numéro de dépôt: 19702093.6
(22) Date de dépôt: 31.01.2019
(51) Int. Cl.: A61Q 19/08, A61K 8/9789

(54) **EXTRAITS DE FRUIT DE MYRCIARIA DUBIA RICHE EN ACIDES ORGANIQUES, COMPOSITIONS COSMETIQUES LE COMPRENANT ET LEURS UTILISATIONS COSMÉTIQUES**
EXTRAKTE AUS MYRCIARIA DUBIA-FRÜCHTEN, DIE REICH AN ORGANISCHEN SÄUREN SIND, KOSMETISCHE ZUSAMMENSETZUNGEN DAMIT UND KOSMETISCHE VERWENDUNGEN DAVON
MYRCIARIA DUBIA FRUIT EXTRACTS RICH IN ORGANIC ACIDS, COSMETIC COMPOSITIONS COMPRISING SAME AND COSMETIC USES THEREOF

(43) Date de publication de la demande: 09.12.2020
(73) Titulaire: ISP Investments LLC, Wilmington, Delaware 19805 (US); Jafer Enterprises R&D, S.L. (Sociedad Unipersonal), 08403 Granollers (Barcelona) (ES)
(72) Inventeur: BOTTO, Jean-Marie, 06560 VALBONNE (FR); CAPALLERE, Christophe, 06200 NICE (FR); GONDRAN, Catherine, 03330 BELLENAVES (FR); IMBERT, Isabelle, 06400 CANNES (FR); GARNIER, Sébastien, 06650 LE ROURET (FR); LE BORGNE, Erell, 06250 MOUGINS (FR)
(74) Mandataire: Axe PI
(86) Numéro de dépôt international: PCT/EP2019/052452
(87) Numéro de publication internationale: WO 2019/149864

(56) Documents cités:
- WO-A1-2014/020219
- FR-A1- 3 049 461
- SERGIO M ZAPATA ET AL: "Camu-camuMyrciaria dubia (HBK) McVaugh: Chemical composition of fruit", JOURNAL OF THE SCIENCE OF FOOD AND AGRICULTURE, vol. 61, no. 3, 1 janvier 1993 (1993-01-01), pages 349-351, XP055523153, GB ISSN: 0022-5142, DOI: 10.1002/jsfa.2740610310
- ALINE SOUZA ET AL: "Bioactive compounds in the peel of camu camu genotypes from Embrapa's active germplasm bank", CIÊNCIA E TECNOLOGIA DE ALIMENTOS, vol. 38, no. 1, 26 octobre 2017 (2017-10-26), pages 67-71, XP055523499, BR ISSN: 0101-2061, DOI: 10.1590/1678-457x.33716
- CHIRINOS R ET AL: "Antioxidant compounds and antioxidant capacity of Peruvian camu camu (Myrciaria dubia (H.B.K.) McVaugh) fruit at different maturity stages", FOOD CHEMISTRY, ELSEVIER LTD, NL, vol. 120, no. 4, 15 juin 2010 (2010-06-15) , pages 1019-1024, XP026877593, ISSN: 0308-8146 [extrait le 2009-11-26]
- PAUL C. LANGLEY ET AL: "Antioxidant and Associated Capacities of Camu Camu ( Myrciaria dubia ): A Systematic Review", JOURNAL OF ALTERNATIVE AND COMPLEMENTARY MEDICINE, vol. 21, no. 1, 1 janvier 2015 (2015-01-01), pages 8-14, XP055523549, US ISSN: 1075-5535, DOI: 10.1089/acm.2014.0130
- JURATE JOCIENE ET AL: "Abstract", APPLIED RESEARCH IN HEALTH AND SOCIAL SCIENCES: INTERFACE AND INTERACTION, vol. 13, no. 1, 1 décembre 2016 (2016-12-01), pages 40-53, XP055523838, ISSN: 1822-3338, DOI: 10.1515/arhss-2016-0005

## Description

L'invention concerne un extrait de fruit de *Myrciaria dubia* (Camu-camu) riche en acides organiques, son procédé de préparation, des compositions cosmétiques le comprenant et leurs utilisations cosmétiques.

Le Camu-camu (*Myrciaria dubia* (H.B.K.) Mc Vaugh.) est un arbuste originaire du bassin amazonien appartenant à la famille des Myrtacées. Cet arbuste d'environ 3 mètres de hauteur croît dans la forêt amazonienne du Pérou, de la Colombie, du Venezuela et du Brésil dans les plaines inondables, lacs et rivières de la majeure partie de l'Amazonie. La floraison de cet arbuste très ramifié a lieu de novembre à décembre et d'août à septembre et la fructification de janvier à mars et d'octobre à novembre.

Les fruits de Camu-camu sont des baies rondes de couleur rouge foncé-violet de 1 à 3 cm de diamètre dont la peau fine est lisse et brillante et la pulpe charnue et acide. Les baies mûres sont ramassées traditionnellement à la main directement en canoë durant les périodes d'inondation de la saison des pluies dans les zones marécageuses amazoniennes. Depuis plusieurs années, des plantations ont vu le jour dans certains pays d'Amazonie. Les peuples autochtones utilisent le Camu-camu à des fins médicinales depuis plus de 700 ans pour traiter les infections, diminuer la douleur, renforcer les articulations, maintenir la longévité, renforcer le système immunitaire et nerveux et faciliter l'absorption des nutriments. Due à son acidité naturelle, la pulpe de ce fruit nutritif est principalement consommée après transformation en jus, boissons alcoolisées, vinaigres, concentrés, confitures, glaces et gâteaux. La pulpe constitue entre 50 et 55 *%* du poids du fruit, le reste étant des graines (1 à 3 graines par fruit) et de la peau. Au niveau mondial, les fruits de Camu-camu ont gagné en popularité lorsque des chercheurs découvrirent les avantages uniques de sa valeur nutritionnelle et des bienfaits sur la santé dus principalement à sa forte teneur naturelle en vitamine C (acide ascorbique) et en flavonoïdes. Le fruit de Camu-camu est connu de l'homme de l'art comme appartenant au groupe des « superfruits » grâce à sa forte richesse en acide ascorbique ou vitamine C (entre 3,5 *%* et 9 *%* du poids sec), en multiples polyphénols, tels que flavonoïdes (myricétine, proanthocyanidine, acide ellagique, etc.), tanins (ellagitanin, essentiellement présent dans les graines) ou anthocyanes, ainsi qu'en composés terpènes (alpha-pinène, limonène, caroténoïde, etc.), ce qui lui procure de forts pouvoirs antioxydants (protection contre le stress oxydatif causé par les radicaux libres) et d'autres vertus pour la santé. Grâce à plusieurs de ces éléments actifs, il est également censé jouer un rôle anti-inflammatoire, renforcer le système immunitaire et améliorer les défenses naturelles de l'organisme. Il est indiqué comme adjuvant en cas de convalescence, de fatigue, de grippe ou d'autres affections virales.

Il est connu¹⁻⁴ que les graines du fruit Camu-camu contiennent de l'acide bétulinique (triterpène), des composés polyphénoliques (myricétine et dérivés, acide ellagique et dérivés, ellagitannins, proanthocyanidines) et des acylphloroglucinols (isomyrtucommulone B, myrciarone B). Il est connu^{1,3-6} également que la peau du fruit Camu-camu contient des composés polyphénoliques (myricétine et dérivés, acide ellagique et dérivés, ellagitannins, proanthocyanidines, cyanidine-3-glucoside, delphinidine-3-glucoside), des caroténoïdes (all*trans* lutéine, beta-carotène, violaxanthine, lutéoxanthine) et des acylpholoroglucinols (myrciarone A, rhodomyrtone). Enfin, il est connu^{4,7-9} que la pulpe du fruit Camu-camu contient des composés polyphénoliques (myricétine et dérivés, acide ellagique et dérivés, ellagitannins, cyanidine-3- glucoside, quercétine et dérivés), de l'acide ascorbique et acide déshydroascorbique, des acides gras (acide stéarique, acide linoléique, acide oléique, acide gamma et alphalinolénique, acide tricosanoïque, acide eicosadienoïque), de l'acide citrique, acide malique et acide isocitrique, des carbohydrates simples (principalement du glucose et du fructose), des acides aminés (sérine, leucine, valine mais aussi glutamate, 4-aminobutanoate, proline, phénylalanine, thréonine, alanine) et des minéraux (potassium, calcium, magnésium, sodium, phosphate, sulfate, aluminium, bore, cuivre, manganèse, zinc). Le document de *Fracassetti et al.*⁴ a également mis en évidence la présence de vitamine C et de polyphénols dans un extrait obtenu à partir d'un mélange de graines et de peau, ou de pulpe, avec un solvant 50 *%* méthanoleau et 1 *%* acide formique, et les quantités obtenues de vitamine C et de polyphénols sont exprimées par rapport à la plante entière.

La concentration des différents ingrédients du fruit de Camu-camu donnée ci-avant a été étudiée à des stades de maturité différente du fruit, soit à partir de matière végétale fraîche ou sèche. *Zapata et al.*⁹ ont étudié la composition chimique de la pulpe fraîche à trois stades différents de maturité. Ils ont ainsi montré que la teneur en vitamine C augmentait d'environ 12 *%* entre le fruit vert et le fruit mûr et que la teneur en sucres (glucose et fructose) était multipliée par deux. La teneur en acides aminés augmente également lors de la maturation du fruit, principalement la teneur en sérine, valine et leucine. Au cours de cette étude, il a également été montré que la teneur en acide citrique diminuait au cours de la maturation, contrairement à l'acide malique dont la teneur augmentait. L'acide isocitrique serait présent en traces dans la pulpe de ce fruit. Pour finir, cette étude a également montré l'augmentation de la teneur en potassium et phosphate, une réduction nette de 50-60 *%* des teneurs en sodium et en sulfate et des teneurs constantes en calcium et magnésium au cours de la maturation.

Parmi les documents cités précédemment, seuls les documents 1 et 4 décrivent l'utilisation du fruit séché de Camu-camu, souvent avec les graines, pour effectuer les analyses des ingrédients. Ces deux documents, utilisant des méthodes d'analyse différentes et des solvants différents, montrent que les extraits présentent des teneurs en polyphénols élevées.

Par ailleurs, le document publié sous le numéro WO2014020219 divulgue des compositions antioxydantes d'un produit obtenu à partir du fruit de Camu-camu. Il concerne plus particulièrement un procédé d'obtention d'un extrait du fruit de Camu-camu à un état de maturation compris entre 20 et 30 *%* et présentant un rapport de matières solides solubles/acidité compris entre 1,40 et 2,30, comprenant des étapes d'élimination des graines des fruits sélectionnés et de séchage des fruits sans graines à une température inférieure à 60 °C. Les composés hydrosolubles sont extraits à l'eau à pH acide (acide tricarboxylique) et il a été démontré que l'extrait contient de grandes quantités de vitamine C et de polyphénols dont les proanthocyanidines. Ces extraits sont décrits pour préparer principalement des compositions alimentaires nourrissantes et des compositions pharmaceutiques stimulantes, les compositions cosmétiques à priori nourrissantes sont citées de manière générale.

On connaît également du document publié sous le numéro WO2004074304 un composé dérivé glycosylé de l'acide gallique (1- [1, 5- dihydroxy- 2, 2, 4, 4- tetraméthyl- 3- [[6- *O*- (3, 4, 5- trihydroxybenzoyl) - β- D- glucopyranosyle] oxy] cyclopentyl] - 3- méthyl- 2- buten-1- one) dénommé « CA1 » issu, selon l'exemple 1, des graines de Camu-camu et possédant une puissante activité antioxydante et un effet blanchissant stable. Le procédé d'extraction est effectué avec du méthanol, un solvant mono-alcoolique, sur les graines de Camu-camu. Selon ce document, le composé CA1 est présent également dans la pulpe et la peau, mais semble-t-il à des concentrations différentes.

Enfin, on connaît du document publié sous le numéro JP3431383 un extrait de Camu-camu présentant également une activité blanchissante. L'extrait est obtenu à partir du fruit entier de Camu-camu, de préférence frais, par macération avec de l'eau, un solvant organique ou leur mélange, pendant une durée d'au moins deux jours. Le seul exemple de ce document indique utiliser une macération du fruit avec de l'éthanol pendant trois jours.

Ainsi, selon la partie du fruit de *Myrciaria dubia* utilisée, la qualité du fruit (fruit frais ou séché) et le procédé d'extraction mis en œuvre, notamment le choix du solvant, les molécules extraites s'avèrent être différentes en qualité et quantité. Or la composition de l'extrait conditionne l'activité biologique et en conséquence l'efficacité cosmétique. De ce fait, les Demandeurs ont souhaité obtenir un nouvel extrait de *Myrciaria dubia* riches en acides organiques et pauvre en polyphénols.

Les Demandeurs ont mis en évidence qu'un extrait de peau et de pulpe du fruit séché de *Myrciaria dubia* présentant une maturité d'au moins 70 % et dépourvus de graines présente une activité réductrice des signes de fatigue de la peau. L'extrait selon l'invention est riche en acides organiques (mélange d'acide ascorbique, acide déshydroascorbique, acide malique et acide citrique), et comprend des minéraux et des sucres (fructose, glucose, saccharose et traces de maltose) et comprend des traces de composés dépigmentant et des composés phénoliques en faible quantité.

L'invention a donc pour premier objet un extrait de pulpe et de peau du fruit séché de Myrciaria dubia dépourvu de graines dont la maturité est d'au moins 70 *%* et dont le ratio de matières solides solubles/acidité est compris entre 2,5 et 3, caractérisé en ce qu'il est riche en acides organiques et en ce qu'il est obtenu à partir de la pulpe et de la peau du fruit séché de *Myrciaria dubia* par extraction solide-liquide dans un solvant hydro-polyalcoolique choisi parmi un solvant hydro-glycérolique et/ou hydro-glycolique, sous agitation classique ou avec ultrasons.

L'invention a pour deuxième objet un procédé d'obtention d'un tel extrait comprenant les étapes suivantes selon lesquelles :
a) on met en dispersion et on extrait dans un solvant hydro-polyalcoolique la pulpe et la peau du fruit séché de *Myrciaria dubia* dépourvu de graines dont la maturité est d'au moins 70 *%* et dont le ratio de matières solides solubles/acidité est compris entre 2,5 et 3 ;
b) on sépare la phase liquide chargée en composés d'intérêt de la phase solide par décantation et/ou centrifugation et/ou filtration clarifiante de l'extrait obtenu suite à l'étape a),
c) on effectue une microfiltration de l'extrait liquide obtenu à l'étape b) pour éliminer les particules solides végétales,
d) éventuellement on purifie l'extrait liquide obtenu à l'étape c) par filtration, ultrafiltration et/ou nanofiltration,
e) éventuellement on peut sécher l'extrait de *Myrciaria dubia* liquide obtenu par exemple par atomisation, lyophilisation ou zéodratation, de manière à obtenir un extrait de *Myrciaria dubia* solide avec éventuellement un support de séchage tel que de la maltodextrine.

L'invention a encore pour objet un extrait de pulpe et de peau de *Myrciaria dubia* obtenu directement par le procédé.

L'invention a pour troisième objet une composition cosmétique comprenant à titre d'agent actif, une quantité efficace d'un extrait de pulpe et de peau du fruit séché de *Myrciaria dubia* selon l'invention, et un milieu physiologiquement acceptable.

Enfin, l'invention a pour quatrième objet l'utilisation cosmétique d'une composition selon l'invention, pour réduire les signes de fatigue de la peau.

Dans cette description, à moins qu'il n'en soit spécifié autrement, il est entendu que, lorsqu'un intervalle est donné, il inclut les bornes supérieures et inférieures dudit intervalle.

Sauf mention contraire, les pourcentages sont des pourcentages massiques.

Dans la présente invention, on entend par :
- « hydro-polyalcoolique » un mélange d'eau et de polyols, ces derniers comprenant au moins deux radicaux alcool, plus précisément de 2 à 9 radicaux alcool et de 3 à 12 atomes de carbone.
- « extrait riche en acides organiques » un extrait contenant une quantité d'acides organiques (mélange d'acide ascorbique total dont acide ascorbique et acide déshydroascorbique, d'acide malique et d'acide citrique) supérieure aux autres ingrédients identifiés, soit entre 13,5 *%* et 80,5 *%* par rapport à la matière sèche de l'extrait total.
- « quantité efficace » la quantité nécessaire de composés d'intérêt pour obtenir le résultat recherché, à savoir, permettre notamment d'obtenir une réduction des signes de fatigue de la peau.
- « réduction des signes de fatigue de la peau », un, plusieurs ou l'ensemble des signes suivants : la présence de poches et de cernes sous les yeux, le teint terne, la sécheresse de la peau, l'accentuation des ridules et rides telles que les rides du front ou de la patte d'oie, l'accentuation du relâchement de la peau, un accroissement de la fragilité des ongles et une réduction de la ligne des cheveux, pouvant apparaitre chez une personne saine. Au niveau moléculaire et cellulaire, la fatigue de la peau se caractérise notamment par un dysfonctionnement mitochondrial, une diminution de la production d'ATP, une diminution de la glycolyse et du système de la créatine kinase.
- Les noms de plantes Camu-camu et *Myrciaria dubia* sont utilisés indifféremment dans la demande.
- « application topique », le fait d'appliquer ou d'étaler une composition contenant l'extrait de *Myrciaria dubia* selon l'invention, à la surface de la peau, d'une muqueuse ou des phanères.
- « physiologiquement acceptable » que les compositions comprenant l'extrait de *Myrciaria dubia* selon l'invention sont appropriées pour entrer en contact avec la peau ou une muqueuse sans provoquer de réactions de toxicité ou d'intolérance ou d'allergie. Dans le cas de compositions pouvant être ingérées ou injectées dans la peau, ces dernières ne présentent pas de toxicité, d'incompatibilité, d'instabilité, ou ne provoquent pas de réponse allergique systémique.
- « matière sèche », exprimée en pourcentage massique, le résidu sec obtenu après élimination totale du solvant par dessiccation ou évaporation.
- « composés d'intérêt », les composés extraits par le procédé selon l'invention et utiles dans une composition cosmétique pour réduire les signes de fatigue de la peau.
- « rapport de matières solides solubles/acidité » est le rapport des matières solides solubles contenues dans le fruit, essentiellement les sucres, sur le taux d'acidité du fruit. Durant le processus de maturation des fruits, les acides sont dégradés, la teneur en sucre augmente et le rapport solides solubles totaux/acidité, nommé Brix/acidité, atteint une valeur plus élevée. Ce rapport n'a pas d'unité et correspond au rapport Brix / acidité calculé sur la base de la concentration de l'acide citrique. La référence peut être trouvée pour le calcul de ce rapport dans le document "Quality assurance in Tropical Fruit Processing" de A. Askar et H. Treptow édité en 1993 par Springer-Verlag Berlin Heidelberg, page 25 paragraphe 2.7.
- « maturité d'au moins 70 *%* » que chaque fruit présente une couleur d'au moins 70 *%* rouge et 30 % verte. Cette maturité pouvant aller jusqu'à 100 *%*.
- « traces de composés » des composés dont la présence est détectable mais non quantifiable avec des méthodes de détection utilisées en routine dans les laboratoires.

La demanderesse a déposé une demande d'accès de la ressource génétique *Myrciaria dubia* (Kunth) McVau auprès du gouvernement Péruvien (INIA : Instituto Nacional de Innovacion Agraria) en conformité avec la loi Andine, Décision no 391, portant sur le régime commun de l'accès aux ressources génétiques.

Le premier objet de l'invention concerne un extrait de pulpe et de peau du fruit séché de *Myrciaria dubia* dépourvu de graines dont la maturité est d'au moins 70 *%* et dont le rapport de matières solides solubles/acidité est compris entre 2,5 et 3, ledit extrait étant riche en acides organiques.

L'invention concerne également un extrait de pulpe et de la peau du fruit séché de *Myrciaria dubia* dépourvu de graines dont la maturité est d'au moins 70 *%* et dont le rapport de matières solides solubles/acidité est compris entre 2,5 et 3, obtenu directement par le procédé décrit comme le 2ème objet de l'invention.

Dans le cadre de la présente invention, la partie de plante choisie est le fruit de *Myrciaria dubia*, dans le groupe constitué par la peau et la pulpe du fruit séché, les graines étant éliminées. Les fruits sont récoltés lorsque leur maturité a atteint 70 *%* ou plus de fruits rouges et que le ratio de matières solides solubles/acidité est compris entre 2,5 et 3.

Dans un mode de réalisation très préférentiel, l'extrait est obtenu de la pulpe et de la peau du fruit séché de *Myrciaria dubia* par extraction solide-liquide dans un solvant hydro-polyalcoolique choisi parmi un solvant hydro-glycérolique et/ou hydro-glycolique, sous agitation classique ou avec ultrasons.

L'extrait est obtenu par extraction solide-liquide de la pulpe et de la peau du fruit séché et broyé de *Myrciaria dubia* dans un solvant hydro-polyalcoolique choisi dans le groupe constitué par l'eau et les polyalcools tels que le glycérol, les glycols tels que le 1,3-propanediol et leurs mélanges. Le solvant 1,3-propanediol est le solvant préféré car ce solvant est agrosourcé.

Le solvant d'extraction est de préférence un solvant hydro-glycérolique et/ou hydro-glycolique, avantageusement dans une proportion comprise entre 30 et 90 *%* de glycérol et/ou de glycol dans l'eau, tel qu'un mélange eau-1,3-propanediol.

Dans un mode de réalisation plus préférentiel encore, l'extrait est obtenu de la pulpe et de la peau du fruit égrainé et séché de *Myrciaria dubia* par extraction solide-liquide, dans un solvant hydro-polyalcoolique composé de 60 *%* de 1,3-propanediol et 40 % d'eau déminéralisée.

Enfin, dans un autre mode de réalisation très préférentiel, l'extrait comprend par rapport à la matière sèche de l'extrait total entre 13,5 *%* et 80,5 % d'acides organiques dont les acides malique, citrique et ascorbique ; entre 4,0 *%* et 22,0 *%* de sucres totaux dont notamment le glucose, le fructose, le saccharose et le maltose ; entre 0,5 *%* et 3 *%* de minéraux dont potassium, magnésium, calcium, sodium, manganèse, zinc et fer.

L'extrait selon l'invention contient seulement des traces de protéines, des traces d'acidesaminés et peptides, une quantité de polyphénols comprise entre 0,08 *%* et 2,00 *%*, et enfin des traces du composé CA1 dépigmentant, pour faire référence aux extraits connus de l'art antérieur.

L'invention a pour deuxième objet un procédé d'obtention d'un extrait selon l'invention comprenant les étapes suivantes selon lesquelles :
a) on met en dispersion et on extrait dans un solvant hydro-polyalcoolique la pulpe et la peau du fruit séché de *Myrciaria dubia* dépourvu de graines dont la maturité est d'au moins 70 *%* et dont le ratio de matières solides solubles/acidité est compris entre 2,5 et 3 ;
b) on sépare la phase liquide chargée en composés d'intérêt de la phase solide par décantation et/ou centrifugation et/ou filtration clarifiante de l'extrait obtenu suite à l'étape a),
c) on effectue une microfiltration de l'extrait liquide obtenu à l'étape b) pour éliminer les particules solides végétales,
d) éventuellement on purifie l'extrait liquide obtenu à l'étape c) par filtration, ultrafiltration et/ou nanofiltration,
e) éventuellement on peut sécher l'extrait de *Myrciaria dubia* liquide obtenu par exemple par atomisation, lyophilisation ou zéodratation, de manière à obtenir un extrait de *Myrciaria dubia* solide avec éventuellement un support de séchage tel que de la maltodextrine.

Pour réaliser le procédé de la présente invention, la partie de plante choisie est le fruit de *Myrciaria dubia*, dans le groupe constitué par la pulpe et la peau du fruit séchées et les graines sont éliminées pour éviter d'extraire des lipides qui compromettraient l'extraction des composés d'intérêt.

Les fruits sont récoltés lorsque leur maturité a atteint 70 *%* ou plus de fruits rouges et que le ratio de matières solides solubles/acidité est compris entre 2,5 et 3.

De manière particulièrement avantageuse, l'étape a) s'effectue sur la pulpe et la peau séchées et broyées du fruit de *Myrciaria dubia* dépourvu de graines. Les fruits récoltés sont triés, désinfectés, lavés et égrainés avant d'être déshydratés à l'air chaud puis la peau et la pulpe sèches sont broyées.

L'extrait est obtenu par extraction solide-liquide de la pulpe et de la peau du fruit séchées et broyées de *Myrciaria dubia* dans un solvant hydro-polyalcoolique choisi dans le groupe constitué par l'eau et les polyalcools tels que le glycérol, les glycols tels que le 1,3-propanediol et leurs mélanges. Le solvant 1,3-propanediol est le solvant préféré car ce solvant est agrosourcé.

Avantageusement, le solvant hydro-polyalcoolique est un mélange comprenant de 10 *%* à 90 *%* en poids de glycérol et/ou de 1,3-propanediol dans de l'eau déminéralisée par rapport au poids total solvant plus eau.

Préférentiellement encore, le solvant hydro-polyalcoolique est un mélange comprenant de 50 *%* à 80 *%* en poids de glycérol et/ou de 1,3-propanediol dans de l'eau déminéralisée par rapport au poids total solvant plus eau.

Plus particulièrement, dans le cadre de l'extraction solide-liquide de la pulpe et peau du fruit égrainé et séché de *Myrciaria dubia*, on introduit entre 0,1 et 20 *%* en poids (matière sèche) de partie de pulpe et peau du fruit dans le solvant d'extraction, et préférentiellement entre 1 et 15 *%* en poids, typiquement entre 5 et 10 *%* en poids (les *%* étant exprimés en poids de la matière sèche par rapport au poids total mis en œuvre).

Lorsque le solvant utilisé est le glycérol, on choisit une proportion comprise entre 30 et 90 *%* de glycérol dans l'eau, et avantageusement entre 50 et 80 *%* (les *%* sont exprimés en poids de glycérol par rapport au poids total eau + glycérol).

Lorsque le solvant utilisé est le glycol et plus particulièrement le 1,3-propanediol, on choisit une proportion comprise entre 10 et 90 *%* de 1,3-propanediol dans l'eau, avantageusement entre 50 et 70 *%* et encore plus avantageusement on choisit une proportion de 60*%* de 1,3-propanediol et 40 % d'eau déminéralisée (les *%* sont exprimés en poids de 1,3-propanediol par rapport au poids total eau plus 1,3-propanediol).

La température d'extraction est avantageusement comprise entre 4°C et 100°C, et préférentiellement entre 10°C et 80°C, et plus particulièrement encore entre 20°C et 50°C.

Le chauffage peut être assuré par circulation d'un fluide thermique dans un récipient d'extraction à double enveloppe ou par micro-ondes.

La durée d'extraction varie avantageusement de 10 minutes à 4 heures, et préférentiellement de 30 minutes à 3 heures, et plus avantageusement encore de 1 à 2 heures.

L'extraction peut être réalisée avantageusement sous vide, à pression atmosphérique ou sous pression, préférentiellement à pression atmosphérique ou sous pression, et avantageusement encore à pression atmosphérique.

L'extraction s'effectue toujours sous agitation permettant ainsi une dispersion et une homogénéisation du solide dans le liquide, améliorant efficacement la diffusion du soluté dans le solvant.

A l'issue de l'extraction de l'étape a), la matière végétale résiduelle et appauvrie en composés d'intérêt est avantageusement séparée de la phase liquide selon l'étape b), par exemple par décantation, par centrifugation ou par filtration clarifiante. La phase liquide obtenue peut être micro-filtrée selon l'étape c) à l'aide de médias filtrants de porosité adaptés afin d'obtenir une solution limpide et exsangue de particules solides végétales.

Ces premières étapes de séparation liquide-solide peuvent-être suivies d'étapes de purification selon l'étape d), par exemple par filtration, ultrafiltration et/ou nanofiltration, permettant de concentrer les composés d'intérêt au dépend d'autres composés.

Ainsi, dans un mode de réalisation préférentiel du procédé selon l'invention, la température de l'étape a) est comprise entre 4°C et 100°C, la durée d'extraction varie de 10 minutes à 4 heures et l'extraction est effectuée sous agitation classique ou avec ultrasons.

L'invention a également pour objet un extrait de pulpe et de peau de *Myrciaria dubia* obtenu directement par le procédé.

L'invention concerne encore un extrait de *Myrciaria dubia* comprenant par rapport à la matière sèche de l'extrait total entre 13,5 *%* et 80,5 % d'acides organiques dont les acides malique, citrique et ascorbique ; entre 4,0 *%* et 22,0 *%* de sucres totaux dont notamment le glucose, le fructose, le saccharose et le maltose ; entre 0,5 *%* et 3 *%* de minéraux dont potassium, magnésium, calcium, sodium, manganèse, zinc et fer et obtenu directement par le procédé ci-dessus.

L'invention concerne encore un extrait de *Myrciaria dubia* comme décrit au paragraphe précédent et comprenant en outre des traces de protéines, des traces d'acides-aminés et peptides, une quantité de polyphénols comprise entre 0,08 et 2 *%* obtenue directement par le procédé ci-dessus.

L'invention a pour troisième objet une composition cosmétique, comprenant un extrait de pulpe et de peau du fruit de *Myrciaria dubia* selon l'invention, et un milieu physiologiquement acceptable.

Dans un mode de réalisation préférentiel selon l'invention, l'extrait hydro-polyalcoolique de la pulpe et de la peau du fruit de *Myrciaria dubia* est présent dans la composition à une concentration de 0,1 à 3 *%* par rapport au poids total de la composition, préférentiellement 0,1 à 1,5 *%*.

Dans un autre mode de réalisation préférentiel, les compositions selon l'invention sont destinées plus particulièrement à une administration par voie topique. Ces compositions doivent donc contenir un milieu physiologiquement acceptable, c'est-à-dire compatible avec la peau et les phanères, et couvrent toutes les formes cosmétiques. Ces compositions pourront notamment être sous forme de crèmes, émulsions huile-dans-eau, ou eau-dans-huile ou émulsions multiples, solutions, suspensions, gels, laits, lotions, sticks ou encore de poudres, et adaptées à une application sur la peau, les lèvres et/ou les phanères. Ces compositions comprennent les excipients nécessaires à leur formulation, tels que solvants, épaississants, diluants, tensioactifs, antioxydants, colorants, conservateurs, parfums. Elles peuvent être utilisées comme produit de soin et/ou comme produit de maquillage de la peau.

Les compositions selon l'invention comprennent, en outre, tout additif communément utilisé dans le domaine d'application envisagé ainsi que les adjuvants nécessaires à leur formulation, tels que des solvants, des épaississants, des diluants, des antioxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

L'INCI Dictionary & Handbook ("International Nomenclature of Cosmetic Ingredients" 13ème Ed. 2010) publié par "the Personal Care Products Council, Inc.", Washington, D.C., décrit une grande variété, sans limitation, d'ingrédients cosmétiques et pharmaceutiques habituellement utilisés dans l'industrie du soin de la peau, qui conviennent pour être utilisés comme ingrédients additionnels dans les compositions selon la présente invention.

Des exemples non limitatifs de ces classes d'ingrédients additionnels incluent : les agents cicatrisants, les agents anti-âge, les agents anti-rides, les agents anti-atrophie, les agents hydratants, les agents adoucissants, les agents antibactériens, les agents antiparasitaires, les agents antifongiques, les agents fongicides, les agents fongistatiques, les agents bactéricides, les agents bactériostatiques, les agents antimicrobiens, les agents anti-inflammatoires, les agents anti-prurigineux, les agents anesthésiques, les agents antiviraux, les agents kératolytiques, les agents anti-radicaux libres, les agents anti-séborrhéiques, les agents anti-pelliculaires, les agents modulant la différenciation, la prolifération ou la pigmentation cutanée, les agents accélérateurs de pénétration, les agents desquamants, les agents stimulant ou inhibant la synthèse de mélanine, les agents blanchissants, dépigmentants, ou éclaircissants, les agents propigmentants, les agents auto bronzants, les agents inhibant la NO-synthase, les agents antioxydants, les agents piégeurs de radicaux libres et/ou anti-pollution atmosphérique, les agents anti-glycation, les agents raffermissants, les agents stimulant la synthèse des macromolécules dermiques ou épidermiques et/ou les agents capables de prévenir ou inhiber leur dégradation, les agents stimulant la synthèse de collagène, les agents stimulant la synthèse d'élastine, les agents stimulant la synthèse de décorine, les agents stimulant la synthèse de laminine, les agents stimulant la synthèse de défensine, les agents stimulant la synthèse de chaperon, les agents stimulant la synthèse de d'aquaporine, les agents stimulant la synthèse d'acide hyaluronique, les agents stimulant la synthèse de lipides et de composants du stratum corneum (céramides, acides gras, ...), les agents inhibant la dégradation du collagène, les agents inhibant la dégradation de l'élastine, les agents stimulant la prolifération des fibroblastes, les agents stimulant la prolifération des kératinocytes, les agents stimulant la prolifération des adipocytes, les agents stimulant la prolifération des mélanocytes, les agents stimulant la différenciation des kératinocytes, les agents stimulant la différenciation des adipocytes, les agents inhibant l'acétylcholinestérase, les agents stimulant la synthèse des glycosaminoglycanes, les agents réparant l'ADN, les agents protégeant l'ADN, les agents anti-démangeaison, les agents pour le traitement et/ou le soin de la peau sensible, les agents raffermissants, les agents anti-vergetures, les agents astringents, les agents régulant la production du sébum, les agents dermo-relaxants, les agents adjuvants de guérison, les agents stimulant la réépithélialisation, les agents adjuvants de réépithélialisation, les facteurs de croissance de cytokine, les agents calmants, les agents anti-inflammatoires, les agents agissant sur la circulation et/ ou microcirculation capillaire, les agents stimulant l'angiogénèse, les agents inhibant la perméabilité vasculaire, les agents agissant sur le métabolisme cellulaire, les agents destinés à améliorer la jonction dermo-épidermique, les agents induisant la pousse des cheveux et/ou des poils, les agents inhibant ou ralentissant la pousse des cheveux et/ou des poils, les agents myorelaxants, les agents anti-pollution et/ou anti-radicalaires, les agents stimulant la lipolyse, les agents amincissants, les agents anti-cellulite, les agents agissant sur la microcirculation, les agents agissant sur le métabolisme des cellules, les agents nettoyants, les agents de coiffage du cheveu, les stimulants de la pousse des cheveux, les écrans solaires, les écrans totaux, les agents de maquillage, les détergents, les produits pharmaceutiques, les agents émulsifiants, les émollients, les solvants organiques, les agents antiseptiques, les actifs déodorants, les milieux physiologiquement acceptables, les surfactants, les agents abrasifs, les absorbants, les composants esthétiques comme les parfums, les pigments, les teintures, colorants et colorants naturels, les huiles essentielles, les agents de toucher, les astringents cosmétiques, les agents anti-acné, les agents anti-coagulation, les agents anti-mousse, les antioxydants, les liguants, les additifs biologiques, les enzymes, les inhibiteurs enzymatiques, les inducteurs enzymatiques, les coenzymes, les agents chélatants, les extraits végétaux et dérivés de plantes, les huiles essentielles, les extraits marins, les agents venant d'un procédé de biofermentation et/ou de biotechnologie, les sels minéraux, les extraits cellulaires, les écrans solaires (les agents photoprotecteurs organiques ou minéraux qui sont actifs contre les rayons ultraviolets A et/ou B) les céramides, les peptides, les tampons, les agents de volume, les agents chélatants, les additifs chimiques, les colorants, les biocides cosmétiques, les dénaturants, les astringents médicinaux, les analgésiques externes, les agents filmogènes, comme les polymères, pour exacerber les propriétés filmogènes et la substantivité de la composition, les dérivés quaternaires, les agents augmentant la substantivité, les agents opacifiants, les ajusteurs et régulateurs de pH (ex. triethanolamine), les propellants, les agents réducteurs, les séquestrants, les agents décolorants et/ou éclaircissants de la peau, les agents conditionnant de la peau (i.e., humectants, incluant miscellanées et occlusifs), les substances retenant l'humidité, les alpha hydroxy acides, les béta hydroxy acides, les hydratants, les enzymes hydrolytiques épidermiques, les agents apaisants et/ou cicatrisants, les agents traitant la peau, les agents anti-rides, les agents capables de réduire ou traiter les poches sous les yeux, les agents exfoliants, les épaississants, les adoucissants, les polymères gélifiants, les vitamines et leurs dérivés, les agents mouillants, les agents pelants, les agents calmants, les agents curatifs de la peau, les lignanes, les conservateurs (i.e.phenoxyethanol et parabens), les anti-UV, les agents cytotoxiques, anti néoplasiques, agents modifiant la viscosité, les solvants non volatils, des agents perlants, les agents antitranspirants, les dépilatoires, vaccins, eau parfumée, agent restructurant la peau, les excipients, les charges, les minerais, les agents anti-mycobactériens, les agents anti-allergéniques, les antihistaminiques Hl ou H2, les anti-irritants, les agents stimulant le système immunitaire, les agents inhibant le système immunitaire, les agents répulsifs d'insectes, les lubrifiants, les pigmentants ou colorants, les agents hypopigmentants, les agents photo-stabilisants, et leurs mélanges, tant qu'ils sont physiquement et chimiquement compatibles avec les autres ingrédients de la composition et spécialement avec les actifs de la présente invention.

Par ailleurs, la nature de ces ingrédients additionnels ne doit pas altérer de manière inacceptable les bénéfices des actifs de l'invention. Ces ingrédients additionnels peuvent être synthétiques ou naturels comme par exemple les extraits de plantes ou venir d'un procédé de biofermentation. Des exemples additionnels peuvent être trouvés dans l'INCI Dictionary & Handbook.

De tels ingrédients additionnels peuvent être choisis dans le groupe comprenant : les sucres aminés, glucosamine, D-glucosamine, N-acetyl- glucosamine, N-acetyl-D-glucosamine, mannosamine, N-acetyl mannosamine, galactosamine, N-acétyl galactosamine, vitamine B3 et ses dérivés, le niacinamide, déhydro-acétate de sodium, l'acide déhydroacétique et ses sels, phytosterols, composés d'acide salicylique, hexamidines, composés dihydroxyproline de dialkanoyl, extraits et dérivés de soja, equol, isoflavones, flavonoïdes, phytantriol, farnesol, géraniol, bisabolol, peptides et leurs dérivés, di -, tri -, tétra -, penta -, et hexapeptides et leurs dérivés, lys-thr-thr-lys-ser, palmitoyl-lys-thr-thr-lys-ser, carnosine, composés d'acide aminé N-acyl, rétinoïdes, propionate de rétinyl, rétinol, palmitate de rétinyl, acétate de rétinyl, rétinal, acide rétinoïque, vitamines hydrosolubles, ascorbates, vitamine C, glucoside ascorbyl, palmitate ascorbyl, magnesium ascorbyl phosphate, sodium ascorbyl phosphate, vitamines et leurs sels et dérivés, provitamines et leurs sels et dérivés, ethyl panthenol, vitamine B et ses dérivés, vitamine B1, vitamine B2, vitamine B6, vitamine B12, vitamine K et ses dérivés, acide pantothénique et ses dérivés, éther éthylique de pantothenyl, panthenol et ses dérivés, panthenol ethylique, dexpanthenol, biotine, acides aminés et leurs sels et les dérivés, acides aminés hydrosolubles, asparagine, alanine, indol, acide glutamique, vitamines insolubles dans l'eau, vitamine A, vitamine E, vitamine F , vitamine D et ses composés, mono-, di -, et triterpénoïdes, bêta-ionol, cedrol, et leurs dérivés, acides aminés insolubles dans l'eau, tyrosine, tryptamine, matériaux particulaires, hydroxytoluène butylé, hydroxyanisole butylé, allantoine, nicotinate de tocophérol, tocophérol, esters de tocophérol, palmitoyl-gly-his-lys, phytostérol, hydroxy acides, acide glycolique, acide lactique, acide lactobionique, kétoacides, acide pyruvique, acide phytique, acide lysophosphatidique, stilbenes, cinnamates, resveratrol, kinétine, zeatin, dimethylaminoethanol, peptides naturels, les peptides de soja, sels de sucres acides, gluconate de manganèse, gluconate de zinc, olamine de piroctone, 3,4,4'- trichlorocarbanilide, triclocarban, pyrithione de zinc, hydroquinone, acide kojique, acide ascorbique, magnésium ascorbyl phosphate, ascorbyl glucoside, pyridoxine, l'aloe vera, les alcools de terpène, allantoine, bisabolol, glycyrrhizinate dipotassique, acide de glycérol, de sorbitol, de pentaerythritol, de pyrrolidone et ses sels, dihydroxyacetone, erythrulose, glycéraldéhyde, tartaraldehyde, l'essence de clou de girofle, le menthol, le camphre, l'essence d'eucalyptus, eugénol, le lactate de menthyle, le distillat d'hamamélis, copolymère d'eicosene et vinyl pyrrolidone, iodopropyl butylcarbamate, un polysaccharide, un acide gras essentiel, un salicylate, un acide glycyrrhétinique, des caroténoïdes, des céramides et des pseudo-céramides, un lipide complexe, les huiles en générale d'origine naturelle telles le beurre de karité, l'huile d'abricot, l'huile d'onagre, l'huile de pruneau, l'huile de palme, l'huile de monoï, l'huile de kahai, hydroquinone, l'HEPES, la procystéine, 1'O-octanoyl-6-D-maltose, le sel disodique d'acide méthyl glycine diacétique, les stéroïdes tels que la diosgénine et les dérivés de la DHEA, la DHEA déhydroépiandrostérone et/ou un précurseur ou dérivé chimique ou biologique, le N-éthylcarbonyl-4-para-aminophénol, les extraits de myrtille, les phytohormones, les extraits de levure *Saccharomyces cerevisiae*, les extraits d'algues, les extraits de soja, de colza, de lin, d'épeautre, de riz, de lupin, de maïs et/ou de pois, l'alvérine et ses sels, en particulier le citrate d'alvérine, les extraits de petits houx et de marron d'inde et leurs combinaisons, un inhibiteur de métalloprotéinases, les extraits de *Schinus molle.*

De tels ingrédients additionnels peuvent être encore être choisis dans le groupe comprenant : des Peptides vendus sous le nom commercial MATRIXYL^{®}, ARGIRELINE^{®}, CHRONOGEN^{™}, LAMINIXYL IS^{™}, PEPTIDE Q10^{™}, COLLAXYL^{™} (brevet FR2827170), PEPTIDE VINCI 01^{™} (brevet FR2837098), PEPTIDE VINCI 02^{™} (brevet FR2841781), ATPeptide^{™} (brevet FR2846883) or un peptide de séquence Arg-Gly-Ser-NH2, vendu sous le nom commercial d'ATPeptide^{™} par ASHLAND^{®}, ou encore un extrait d'Artémia salina, vendu sous le nom commercial de GP4G^{™} (FR2817748), un extrait de levure (Dynagen^{™}, brevet FR2951946 ou Actopontine^{™} brevet FR2944526).

Dans tous les cas, l'homme de métier veillera à ce que ces adjuvants ainsi que leurs proportions soient choisis de telle manière à ne pas nuire aux propriétés avantageuses recherchées de la composition selon l'invention.

Enfin, un quatrième objet de l'invention est l'utilisation de compositions cosmétiques pour réduire les signes de fatigue de la peau, chez une personne saine.

Les signes de fatigue de la peau résultent des effets combinés de plusieurs facteurs en lien avec le style de vie (habitudes alimentaires, exposition à la pollution), les émotions (stress psychologique, surmenage) ainsi que le manque de repos ou un sommeil de mauvaise qualité (Flament et al, How a working day-induced-tiredness may alter some facial signs in differently aged Caucasian women, Int J Cos Sci, 1-9, 2017). Au niveau clinique, les signes de fatigue de la peau les plus souvent observés au niveau du visage sont : la présence de poches et de cernes sous les yeux, le teint terne, la sécheresse de la peau, l'accentuation des ridules et rides telles que les rides du front ou de la patte d'oie, l'accentuation du relâchement de la peau, un accroissement de la fragilité des ongles et une réduction de la ligne des cheveux ont aussi été observés chez des personnes saines, en lien avec la fatigue.

La présente demande porte sur des compositions de soin et des méthodes de soins cosmétiques s'adressant à des personnes saines.

Au niveau moléculaire et cellulaire, un dysfonctionnement mitochondrial a été associé à la fatigue (Filler et al, Association of mitochondrial dysfunction and fatigue : a review of the literature, BBA Clinical, 1, 12-23, 2014). En effet, les mitochondries jouent un rôle essentiel dans la production d'énergie sous forme d'ATP. Le maintien du niveau énergétique dans la cellule est indispensable pour les activités de synthèse, de régénération et division cellulaire ainsi que de protection et de réparation (Blatt et al, Stimulation of skin's energy metabolism provides multiple benefits for mature human skin, Biofactors 25, 179-185, 2005). Dans la cellule, la production d'énergie peut se faire également par des voies extra-mitochondriales telles que la glycolyse et le système de la créatine kinase. La glycolyse représente une voie métabolique clé pour les cellules prolifératives tels que les kératinocytes de la peau. L'enzyme 6-phosphofructo-2-kinase/fructose-2,6-biphosphatase 3 (PFKFB3) joue un rôle important dans la régulation de la glycolyse en catalysant la formation de fructose 2,6 biphosphate, dont l'accumulation favorise l'activité de la phosphofructokinase 1, enzyme jouant un rôle limitant majeur dans la glycolyse (Amelio et al, p63 adjusts sugar taste of epidermal layers, J Invest Dermatol, 137, 1204-1206, 2017). La PFKFB3 est requise pour maintenir l'activité proliférative des kératinocytes (Hamanaka et al, PFKFB3, a direct target of p63, is required for proliferation and inhibits differentiation in epidermal keratinocytes, J Invest Dermatol, 137, 1267-1276, 2017).

La créatine kinase est aussi une enzyme clé de la capacité énergétique de la cellule. L'ATP est stocké sous la forme de phosphocréatine, produite par la créatine kinase, ce qui permet ensuite son transport et sa disponibilité en cas de forte demande énergétique (Lenz et al, The creatine kinase system in human skin : protective effects of creatine against oxidative and UV damage in vitro and in vivo, J Invest Dermatol, 124 :443-452, 2005).

Ainsi, l'invention a pour objet l'utilisation cosmétique d'une composition selon l'invention pour réduire les signes de fatigue pouvant apparaître chez une personne saine, dans laquelle les signes de fatigue de la peau sont choisis parmi : la présence de poches et de cernes sous les yeux, le teint terne, la sécheresse de la peau, l'accentuation des ridules et rides telles que les rides du front ou de la patte d'oie, l'accentuation du relâchement de la peau, un accroissement de la fragilité des ongles et une réduction de la ligne des cheveux, ou l'ensemble de ces signes.

L'invention a encore pour objet l'utilisation cosmétique d'une composition dans laquelle les signes de fatigue de la peau se manifestent au niveau moléculaire et cellulaire, par une diminution de la production d'ATP, de la glycolyse et du système de la créatine kinase, de l'expression du collagène de type I, de la synthèse de PFKFB3, du niveau d'ARN messager (ARNm) de 11b-HSD2 et une augmentation du niveau d'ARN messager (ARNm) de 11b-HSD1.

L'invention a encore pour objet l'utilisation cosmétique d'une composition selon l'invention pour augmenter la synthèse d'ATP cellulaire, augmenter l'activité de la créatine kinase, augmenter l'expression du collagène de type I, augmenter la synthèse de PFKFB3, diminuer le niveau d'ARN messager (ARNm) de 11b-HSD1 et augmenter le niveau d'ARN messager (ARNm) de 11b-HSD2.

L'invention et les avantages qui en découlent seront mieux compris à la lecture de la description et des modes de réalisation non limitatifs qui suivent, rédigés au regard des figures annexées dans lesquelles :
- La figure 1 représente la mesure d'ATP sur des cultures de fibroblastes dermiques
- La figure 2 représente la mesure de l'activité de la créatine kinase sur des cultures de fibroblastes dermiques
- La figure 3 représente la mesure par qPCR du collagène de type COL1A1 sur derme reconstruit
- La figure 4 représente la mesure par qPCR de 11b-HSD1 et 11b-HSD2 sur des cultures de kératinocytes humains normaux (KHN)
- La figure 5 représente la coloration hématoxyline/ éosine (HE) sur épiderme reconstruit
- La figure 6 représente l'intensité de l'immunomarquage du PFKFB3 sur épiderme reconstruit
- La figure 7 représente la mesure de l'activité de la créatine kinase sur épiderme reconstruit

### Exemple 1 : Préparation d'un extrait végétal à partir de poudre de pulpe et de peau du fruit Myrciaria dubia égrainé

La pulpe et la peau du fruit frais égrainé de *Myrciaria dubia* de maturité 70 *%* sont placées pour séchage dans un système de tunnel à air chaud chauffé à 60°C sous contrôle d'humidité. La poudre obtenue (200g) est placée dans un réacteur en verre, muni d'une agitation et d'un chauffage par double enveloppe en présence d'un fluide caloporteur.

Le solvant utilisé (3800 g) pour la macération est un mélange de 60 *%* de 1,3-propanediol et 40 % d'eau déminéralisée.

La poudre de pulpe et de peau de *Myrciaria dubia* est dispersée dans le solvant pendant 2 heures à 30°C puis le mélange est séparé sur décanteur, puis centrifugé afin d'éliminer le culot, et le surnageant est récupéré et filtré. Après filtration, le surnageant est un extrait de 3220 g, en milieu solvant 60 *%* 1,3-propanediol et 40 *%* eau.

L'extrait ainsi obtenu est dénommé « Extrait de *Myrciaria* E1 » dans les exemples suivants. Cet extrait E1 contient 2,0 *%* de matière sèche comportant lui-même (résultats exprimés par rapport à la Matière Sèche (MS)) :
- 35.3 % / MS d'acides organiques dont les acides malique, citrique et acide ascorbique total (acide ascorbique et acide déshydroascorbique) ;
- 10,1 *%* / MS de sucres dont notamment le glucose, le fructose, le saccharose et le maltose ;
- 1,8 *%* / MS de minéraux dont potassium, magnésium, calcium, sodium, manganèse, zinc et fer ; des traces de peptides / protéines et des traces de composés CA1 ;
- 1,5 *%* /MS de polyphénols sous forme ellagique et gallique.

L'extrait sec décrit ci-dessus est obtenu par une méthode gravimétrique basée sur la masse avant et après évaporation du solvant présent dans l'extrait liquide.

L'acide ascorbique total (acide ascorbique + acide déshydroascorbique) a été dosé selon une méthode HPLC/ par calibration externe.

Les acides citrique et malique ont été dosés selon une méthode HPLC/DEDL par calibration externe.

Les sucres totaux ont été dosés selon une méthode HPLC/DEDL par calibration externe.

Les polyphénols ont été dosés par HPLC/DAD par calibration externe.

Les minéraux ont été dosés selon une méthode adaptée de la norme NF EN ISO 11885 (la méthode NF EN ISO 11885 est une méthode analytique par ICP/OES).

Liste des abréviations :
- HPLC : High-Performance Liquid Chromatography (chromatographie liquide haute performance)
- DAD : Diode Array Detector (détecteur à barrettes de diodes)
- DEDL : Détecteur Evaporatif à Diffusion de Lumière
- ICP-OES : Inductively Coupled Plasma Optical Emission Spectrometry (spectrométrie d'émission optique à plasma à couplage inductif)

### Exemple 2 : Effet de l'extrait de Myrciaria E1 sur la synthèse d'ATP sur cultures de fibroblastes dermiques en condition de fatigue

Le but de cette étude est de déterminer le niveau de synthèse d'ATP sur cultures de fibroblastes dermiques « fatiguées » par des réplications et un milieu pauvre en nutriment. Ces cultures sont traitées avec l'extrait E1 à 0,1 % (volume/volume), c'est-à-dire dilué au 1/1000ème dans le milieu de culture.

Protocole : Des cultures de fibroblastes dermiques sont « fatiguées » par quelques réplications (cellules utilisées entre les passages 15 et 20) et sont nourries par un milieu pauvre en nutriment (sans glucose) durant 3 jours. Ces cultures sont traitées avec l'extrait de *Myrciaria* E1 à 0,1 *%*, à partir du 2ème jour de stress et ce pendant les 2 jours de stress restants soit 48h de traitement. Le 4ème jour, une application de l'extrait de *Myrciaria* E1 à 0,1*%* pendant 1 minute est réalisé puis le dosage de l'ATP est effectué par luminescence (Abcam - réf : ab113849).

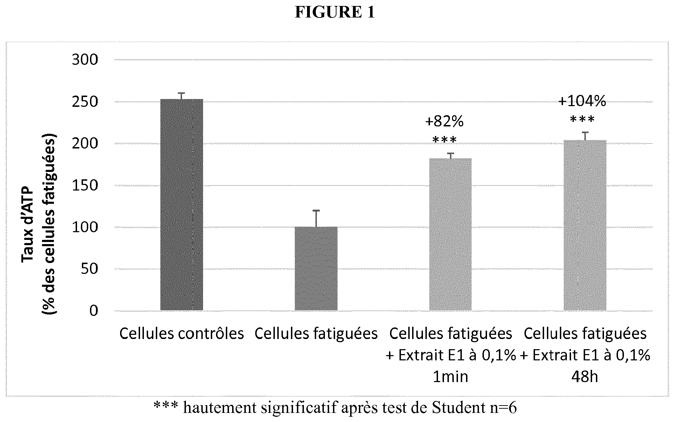

Résultats : Après 48 h de traitement avec l'extrait de *Myrciaria* E1, le niveau d'ATP est augmenté de +104 *%* par rapport aux fibroblastes « fatigués » non traités. Après 1 min de traitement avec l'extrait de *Myrciaria* E1, le niveau d'ATP est augmenté de +82 *%* par rapport aux fibroblastes « fatigués » non traités. Les résultats sont présentés sur la figure 1.

Conclusion : Le traitement des fibroblastes « fatigués » avec l'extrait de *Myrciaria* E1 à 0,1 % conduit à une augmentation de la synthèse d'ATP.

### Exemple 3 : Effet de l'extrait de Myrciaria E1 sur l'activité de la créatine kinase sur cultures de fibroblastes dermiques en condition de fatigue

Le but de cette étude est de déterminer le niveau d'activité de la créatine kinase sur cultures de fibroblastes dermiques. Cette enzyme a pour fonction de catalyser la conversion de la créatine en phosphocréatine, constituant ainsi une réserve d'énergie rapidement utilisable.

Cette mesure d'activité se fait sur des cultures de fibroblastes dermiques « fatiguées » traitées avec l'extrait de *Myrciaria* E1 à 0,1 % (volume/volume), c'est-à-dire dilué au 1/1000ème dans le milieu de culture.

Protocole : Des cultures de fibroblastes dermiques sont « fatiguées » par quelques réplications (cellules utilisées entre les passages 15 et 20) et sont nourries par un milieu pauvre en nutriment (sans glucose) durant 3 jours. Ces cultures sont traitées avec l'extrait de *Myrciaria* E1 à 0,1*%*, ou avec de la vitamine C à 4µg/mL en tant que contrôle positif, à partir du 2ème jour de stress et ce pendant les 2 jours de stress restants. 2 jours après le début du traitement, une mesure de l'activité de la créatine kinase est réalisée à l'aide d'un kit (Abcam - ref : ab155901).

Pour cela, les fibroblastes sont détachés avec le CK buffer contenu dans le kit, récupérés et centrifugés à 4000g pendant 5 min à 4°C. L'activité est ensuite mesurée pour chaque échantillon suivant le protocole du kit. Une cinétique est obtenue par une lecture colorimétrique au spectromètre Synergy 2 (Biotek) couplé au logiciel to Gen5.

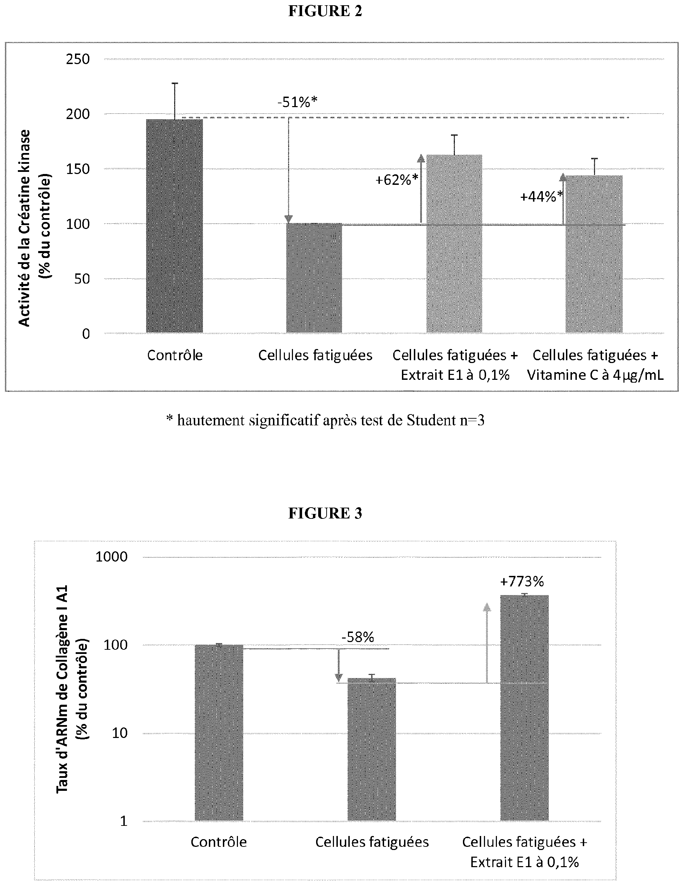

Résultats : Dans la condition où les cellules sont « fatiguées », l'activité de la créatine kinase diminue de -51 *%*. Avec le traitement à 0,1 % d'extrait de *Myrciaria* E1, cette activité enzymatique augmente de +62 *%* par rapport aux cellules « fatiguées » non traitées et de seulement +44 *%* avec le traitement à la vitamine C. Les résultats sont présentés sur la figure 2.

Conclusion : Le traitement des cellules avec l'extrait de *Myrciaria* E1 à 0,1 *%* prévient la diminution de l'activité de la créatine kinase sur cellules fatiguées et est plus important que l'effet observé avec la vitamine C.

### Exemple 4 : Effet de l'extrait Myrciaria E1 sur la mesure par qPCR du collagène de type COL1A1 sur derme reconstruit en condition de fatigue

Le but de cette étude est de déterminer le niveau d'ARN messager (ARNm) de collagène I sur des équivalents de dermes reconstruits à partir de fibroblastes fatigués par des réplications et un milieu pauvre en nutriment. Ces cultures sont traitées avec l'extrait de *Myrciaria* E1 à 0,1 *%* (volume/volume) c'est-à-dire dilué au 1/1000^{ème} dans le milieu de culture.

Protocole : Des cultures de fibroblastes dermiques sont « fatiguées » par quelques réplications (cellules utilisées entre les passages 15 et 20) et sont nourries par un milieu pauvre en nutriment (sans glucose) durant 2 jours. Ces cultures sont également traitées pendant ces 2 jours avec l'extrait de *Myrciaria* E1 à 0,1 *%* (volume/volume), c'est-à-dire dilué au 1/1000ème. Une culture de fibroblastes (passage entre 4 et 10) maintenus dans du milieu DMEM 1g/L en glucose supplémenté avec 10 % (v/v) de sérum de veau fœtal, 2 mM de L-glutamine (Lonza) et 100 µg/mL de Primocine (Invivogen), est utilisé en tant que contrôle.

A partir de ces cultures de fibroblastes et de collagène, des équivalents de derme sont reconstitués. Une fois la reconstitution faite, l'extrait de *Myrciaria* E1 à 0,1 *%* est rajouté une fois à J0, J1 et J2. A J5 les dermes sont congelés à -80°C.

Les dermes sont ensuite décongelés afin d'en extraire les ARN totaux.

Pour cela, les dermes sont broyés à l'aide du Gentle MACS (Mylteni) et les ARN totaux extraits en utilisant le mirVana^{∗} miRNA Isolation Kit (AM1561, Ambion). Les ARN sont ensuite reverse-transcrits avec le High Capacity cDNA Reverse Transcription Kit avec inhibiteur de RNase (4374966, Life technologies). Finalement, la réaction de PCR en temps réel est réalisée sur un thermocyleur StepOnePlus (Applied Biosystems) avec le TaqMan^{∗} Gene Expression Master Mix (4369514, Life technologies) et les TaqMan^{∗} Gene Expression Assays (Life technologies). La méthode comparative des Ct (Cycle Threshold ou « cycle du seuil ») est utilisée pour la quantification relative des cibles et le StepOne^{∗} software (Applied Biosystems) pour le traitement des données.

Résultats : Dans la condition où les fibroblastes sont « fatigués », le niveau en ARN messager (ARNm) de collagène I est diminué par rapport aux fibroblastes non fatigués de -58 *%*. Lorsque ces fibroblastes ont été cultivés avec l'extrait de *Myrciaria* E1 à 0,1 %, le taux d'ARN messager (ARNm) de collagène I augmente de +773 *%*. Les résultats sont présentés sur la figure 3.

Conclusion : Le traitement de fibroblastes « fatigués » avec l'extrait *Myrciaria* E1 à 0,1 *%*, avant et après une reconstitution de derme équivalent conduit à une augmentation des ARN messager (ARNm) de collagène I.

### Exemple 5 : Effet de l'extrait de Myrciaria E1 sur la mesure par qPCR de 11b-HSD1 et 11b-HSD2 sur des cultures de kératinocytes humains normaux (KHN)

Le but de cette étude est de déterminer le niveau d'ARN messager (ARNm) de 11b-HSD1 et 11b-HSD2 sur des kératinocytes. Les enzymes 11b-HSD catalysent l'interconversion du cortisol actif en cortisone inactive. Or, le taux de cortisol a été associé à l'état d'anxiété et de stress psychologique, pouvant contribuer aux signes de fatigue de la peau. Ces cultures sont traitées avec l'extrait de *Myrciaria* E1 à 0,1 % (volume/volume), c'est-à-dire dilué au 1/1000ème dans le milieu de culture.

Protocole : Des kératinocytes sont mis en culture et sont traités avec l'extrait de *Myrciaria* E1 à 0,1 *%*, une fois par jour pendant 24 heures et 48 heures. Les niveaux en ARN messager (ARNm) de 11b-HSD1 et 11b-HSD2 sont mesurés par PCR en temps réel.

Pour cela, les ARN totaux sont extraits en utilisant le mirVana^{∗} miRNA Isolation Kit (AM1561, Ambion) puis reverse-transcrits avec le High Capacity cDNA Reverse Transcription Kit avec inhibiteur de RNase (4374966, Life technologies). Finalement, la réaction de PCR en temps réel est réalisée sur un thermocyleur StepOnePlus (Applied Biosystems) avec le TaqMan^{∗} Gene Expression Master Mix (4369514, Life technologies) et les TaqMan^{∗} Gene Expression Assays (Life technologies). La méthode comparative des Ct (Cycle Threshold ou « cycle du seuil ») est utilisée pour la quantification relative des cibles et le StepOne^{∗} software (Applied Biosystems) pour le traitement des données.

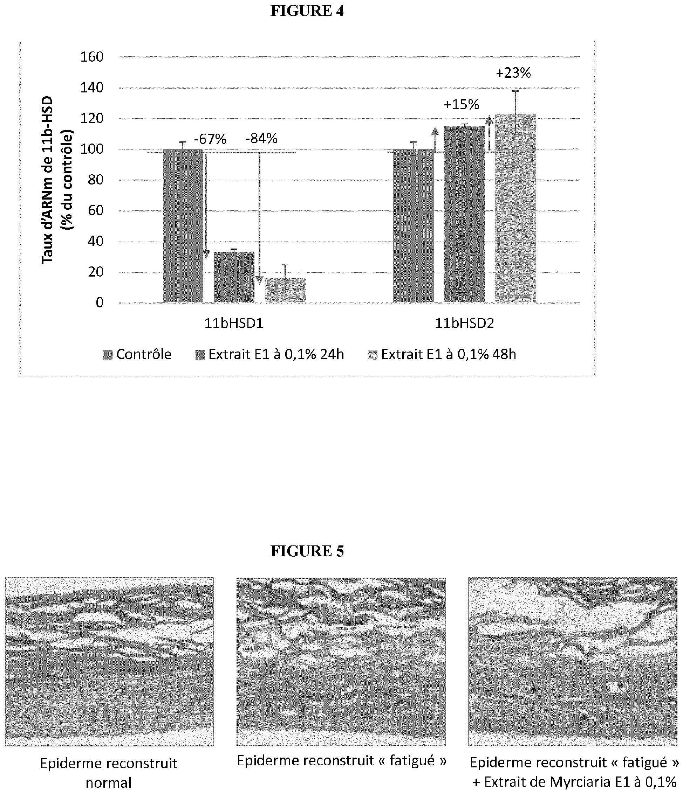

Résultats : Dans les KHN traités par l'extrait de *Myrciaria* E1, le niveau de 11b-HSD1 diminue respectivement de -67 *%* et de -84 *%* après 24h et 48h de traitement. Le niveau de 11b-HSD2 augmente respectivement de +15 *%* et de +23 *%* après 24h et 48h de traitement. Les résultats sont présentés sur la figure 4.

Conclusion : Le traitement de kératinocytes avec l'extrait de *Myrciaria* E1 à 0,1 *%* conduit à une diminution du niveau d'ARN messager (ARNm) de 11b-HSD1 et à une augmentation du niveau d'ARN messager (ARNm) de 11b-HSD2.

### Exemple 6 : Effet de l'extrait de Myrciaria E1 sur la morphologie d'épiderme reconstruit en condition de fatigue

Le but de cette étude est de créer un modèle de peau reconstruite humaine « fatiguée ». Pour cela, la peau est reconstruite en utilisant des kératinocytes de donneur adulte ainsi qu'un inhibiteur de la chaine respiratoire (la roténone) afin de créer un manque d'énergie.

L'utilisation d'un inhibiteur de la chaine respiratoire tel que la roténone crée un dysfonctionnement mitochondrial, incluant une déplétion en ATP. Par conséquent, le manque d'énergie cellulaire a été assimilé dans notre modèle à un état de fatigue cellulaire (Han G et al, The mitochondrial complex I inhibitor rotenone induces endoplasmic reticulum stress and activation of GSK-3β in cultured rat retinal cells, Invest Ophthalmol Vis Sci. 31;55(9):5616-28, 2014). Ces cultures sont traitées pendant la reconstruction avec l'extrait de *Myrciaria* E1 à 0,1 *%* (volume/volume), c'est-à-dire dilué au 1/1000eme ou à 1 *%* (volume/volume), c'est-à-dire dilué au 1/100eme dans le milieu de culture.

Protocole : Au 12^{eme} jour de reconstruction des épidermes, la roténone est ajoutée dans le milieu de culture. Une condition contrôle est maintenue sans ajouter de roténone mais en changeant quand même le milieu de culture. Après 3h d'incubation avec l'inhibiteur, le milieu est changé par un milieu neuf contenant 0,1 % d'extrait de *Myrciaria* E1 et les épidermes sont à nouveau incubés pendant 48h. La condition placebo contient du PBS (phosphate buffer saline) dans le milieu de culture. La morphologie de la peau est ensuite observée par une coloration Hématoxyline-Eosine (HE).

Pour cela, les épidermes reconstruits sont fixés avec du formaldéhyde puis inclus dans de la paraffine. Des coupes de 4 µm sont ensuite réalisées.

Pour la coloration, les coupes sont déparaffinées par des bains de xylène et d'éthanol puis rincées. La coloration se fait par un bain d'hématoxyline puis d'éosine.

Les coupes sont ensuite examinées au microscope (Eclipse E600, Nikon) et les photos sont prises avec le logiciel Q-capture acquisition (QImaging^{∗}).

Résultats : Dans la condition où les épidermes reconstruits sont traités par la roténone, nous observons un épiderme plus fin que celui du contrôle et une couche basale plus endommagée. Lorsque ceux-ci sont traités avec 0,1 % d'extrait de *Myrciaria* E1, l'épiderme semble montrer moins de dommage dans sa structure. Les résultats sont présentés sur la figure 5.

Conclusion : Le traitement des épidermes reconstruits avec l'extrait de *Myrciaria* E1 à 0,1 % semble compenser partiellement le manque d'énergie induit par l'inhibition de la chaine respiratoire par la roténone.

### Exemple 7 : Effet de l'extrait de Myrciaria E1 sur l'expression du PFKFB3 sur un épiderme reconstruit en condition de fatigue

Le but de cette étude est de déterminer le niveau d'expression de la PFKFB3 (6-phosphofructo-2-kinase/fructose-2,6-biphosphatase 3) dans un modèle de peau reconstruite humaine « fatiguée ».

La PFKFB3 intervient dans le processus de glycolyse. C'est une enzyme inductible qui est exprimée par les cellules épithéliales active de l'épiderme. Elle permet l'activation de l'enzyme PFK-1 (phosphofructokinase-1) qui limite ce processus de glycolyse.

Pour obtenir une peau « fatiguée », celle-ci est reconstruite en utilisant des kératinocytes de donneur adulte ainsi qu'un inhibiteur de la chaine respiratoire (la roténone) afin de créer un manque d'énergie (cf. exemple 6). Ces cultures sont traitées pendant la reconstruction avec l'extrait de *Myrciaria* E1 à 0,1 % (volume/volume), c'est-à-dire dilué au 1/1000eme dans le milieu de culture.

Protocole : Au 12^{eme} jour de reconstruction des épidermes, la roténone est ajoutée dans le milieu de culture. Une condition contrôle est maintenue sans ajouter de roténone mais en changeant quand même le milieu de culture. Après 3h d'incubation avec l'inhibiteur, le milieu est changé par un milieu neuf contenant 0,1 % de l'extrait de *Myrciaria* E1 et les épidermes sont à nouveau incubés pendant 48h. La condition placebo contient du PBS (phosphate buffer saline) dans le milieu de culture. Une détection de l'expression de la PFKFB3 est réalisée par une technique d'immunofluorescence indirecte.

Pour cela, les épidermes reconstruits sont fixés avec du formaldéhyde puis inclus dans de la paraffine. Des coupes de 4 µm sont ensuite réalisées.

Pour la coloration, les coupes sont déparaffinées par des bains de xylène et d'éthanol puis rincées. Pour démasquer les sites spécifiques de liaison aux antigènes, les coupes sont chauffées dans un bain de tampon citrate au four à micro-onde puis traitées avec une solution de trypsine. Après une saturation avec de la BSA à 5 *%* pendant 30 minutes pour bloquer les sites non spécifiques, les coupes sont ensuite incubées avec une solution d'anticorps dirigés contre la PFKFB3 pendant une heure et demi, puis avec une solution d'anticorps secondaire anti-lapin couplé à un fluorochrome (Alexa Fluor^{®} 488, Invitrogen) pendant une heure. Les coupes sont alors examinées au microscope à Epi-fluorescence (Zeiss Axiovert 200M microscope). Une quantification de la fluorescence, à l'aide du logiciel Volocity^{®} image analysis software (PerkinElmer, Inc.) est réalisée à partir des photographies obtenues.

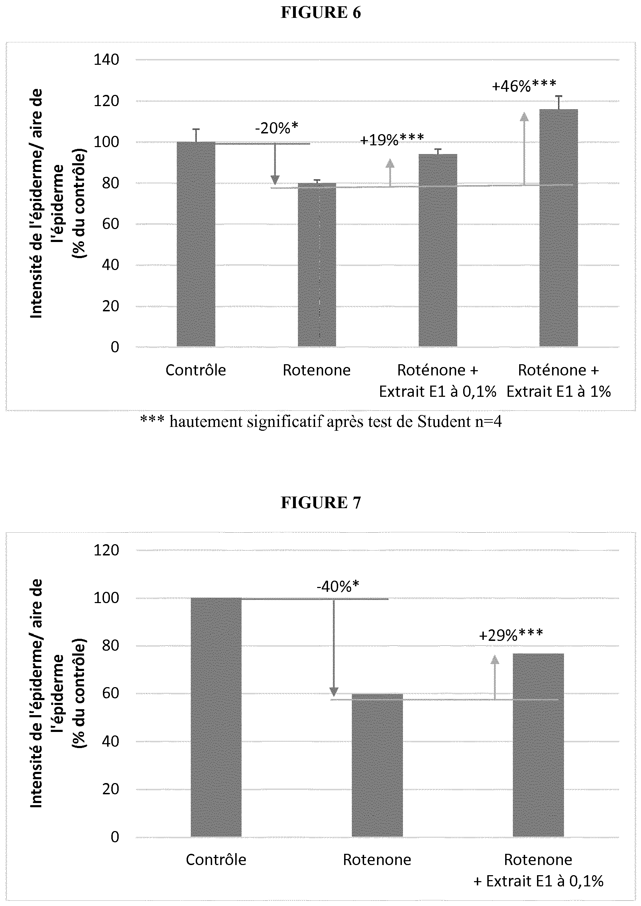

Résultats : Dans la condition où les épidermes reconstruits sont traités par la roténone, nous observons une diminution de l'expression du PFKFB3 de -20 *%* comparé aux épidermes non stressés. Lorsque ceux-ci sont traités avec 0,1 % ou 1% d'extrait de *Myrciaria* E1, son expression augmente respectivement de +19 *%* et +46 *%*. Les résultats sont présentés sur la figure 6.

Conclusion : Les épidermes reconstruits traités avec la roténone montre une diminution de la PFKFB3 traduisant d'une glycolyse amoindrie. Le traitement de ces épidermes reconstruits « fatigués » avec l'extrait de *Myrciaria* E1 à 0,1 *%* semble compenser partiellement ce manque d'énergie observée par une stimulation de la synthèse de la PFKFB3.

### Exemple 8 : Effet de l'extrait de Myrciaria E1 sur l'activité de la créatine kinase sur un épiderme reconstruit en condition de fatigue

Le but de cette étude est de déterminer le niveau d'activité de la créatine kinase dans un modèle de peau reconstruite humaine. Cette enzyme a pour fonction de catalyser la conversion de la créatine en phosphocréatine, constituant ainsi un réservoir d'énergie rapidement utilisable.

Cette mesure d'activité se fait sur un modèle de peau reconstruite humaine « fatiguée » obtenu en utilisant des kératinocytes de donneur adulte ainsi qu'un inhibiteur de la chaine respiratoire (la roténone) afin de créer un manque d'énergie. Ces cultures sont traitées pendant la reconstruction avec l'extrait de *Myrciaria* à 0,1 % (volume/volume), c'est-à-dire dilué au 1/1000eme dans le milieu de culture.

Protocole : Au 12^{eme} jour de reconstruction des épidermes, la roténone est ajoutée dans le milieu de culture. Une condition contrôle est maintenue sans ajouter de roténone mais en changeant quand même le milieu de culture. Après 3h d'incubation avec l'inhibiteur, le milieu est changé par un milieu neuf contenant 0,1 % d'extrait de *Myrciaria* E1 et les épidermes sont à nouveau incubés pendant 48h. La condition placebo contient du PBS (phosphate buffer saline) dans le milieu de culture. Une mesure de l'activité de la créatine kinase est réalisée à l'aide d'un kit (Abcam - ref : ab155901).

Pour cela, les épidermes reconstruits sont broyés à l'aide d'un pilon en verre avec le CK buffer contenu dans le kit, récupérés et centrifugés à 4.000g pendant 5 min à 4°C. L'activité est ensuite mesurée pour chaque échantillon suivant le protocole du kit. Une cinétique est obtenue par une lecture colorimétrique au spectromètre Synergy 2 (Biotek) couplé au logiciel to Gen5.

Résultats : Dans la condition où les épidermes reconstruits sont « fatigués », l'activité de la créatine kinase diminue de -40 *%* comparé aux épidermes non « fatigués ». Avec le traitement à 0,1 % de l'extrait de *Myrciaria* E1, cette activité enzymatique augmente de +29 *%* par rapport au modèle « fatigué ». Les résultats sont présentés sur la figure 7.

Conclusion : Le traitement des épidermes reconstruits avec l'extrait de *Myrciaria* E1 à 0,1 % prévient la diminution de l'activité de la créatine kinase observée sur modèle de peau reconstruite « fatiguée ».

### Exemple 9 : Formulation Crème velours

| Ingrédient / Nom commercial | Nom INCI | *%* |
|---|---|---|
| Phase A | | |
| Eau purifiée | Water | 70,48 |
| Phase B | | |
| Natrosol^{™} Plus 330 CS | Cetyl Hydroxyethylcellulose | 0,50 |
| Solagum AX^{∗} | Acacia Senegal Gum & Xanthan Gum | 1,00 |
| Phase A | | |
| Phase C | | |
| Simulgreen 18-2^{∗} | Hydroxystearyl Alcohol & Hydroxystearyl Glucoside | 3,00 |
| Phase D | | |
| Ceraphyl^{™} 791 ester | Isocetyl Stearoyl Stearate | 5,00 |
| Macadamia Nut Oil CP RBD | Macadamia Ternifolia Seed Oil | 5,00 |
| Virgin Prunus Oil | Prunus Domestica Seed Extract | 2,00 |
| Orchid^{™} Complex OS ester | Caprylic/Capric Triglyceride (and) Cymbidium Grandiflorum Flower Extract | 5,00 |
| Phase E | | |
| Optiphen^{™} BSB-N preservative | Benzyl Alcohol (and) Glycerin (and) Benzoic Acid (and) Sorbic Acid | 1,00 |
| Zemea^{∗} | Propanediol | 5,00 |
| Phase F | | |
| Extrait E1 | 1,3-Propanediol (and) Water/Aqua (and) Myrciaria Dubia Fruit Extract | 1,00 |
| Phase G | | |
| Eau purifiée | Water | 1,00 |
| Sodium Hydroxide | | 0,02 |

Méthode de préparation :
1. Dans le bécher principal, ajouter l'eau et commencer à homogénéiser en chauffant à 70-75°C.
2. Saupoudrer le Natrosol Plus 330 CS et mélanger bien pendant 30 minutes.
3. Saupoudrer le Solagum AX et mélanger bien pendant 20 minutes.
4. Ajouter les ingrédients de la Phase dans un deuxième bécher et chauffer à 70-75°C.
5. Ajouter la Phase C à la Phase A en homogénéisant.
6. A 70-75°C ajouter la Phase D dans le bécher principal et mélanger bien. L'émulsion doit être homogène.
7. Commencer à refroidir. Ajouter les ingrédients de la Phase E un par un à 50°C en mélangeant bien après chaque ajout.
8. A 25°C, ajouter la Phase F et homogénéiser.
9. Prémélanger la Phase G jusqu'à obtenir un mélange clair et uniforme. Ajouter au bécher principal et mélanger bien.
10. Arrêter à 25°C.
11. Apparence : Emulsion blanche ; opaque. pH: 4,4 - 4,8 ; Viscosité (D0) : 12000 - 20000 cps (Brookfield RVT/Spindle B/5 RPM/1 minute/25°C)
12. La conservation de la formule a été validée par un double test d'efficacité après 28 jours. Toutefois, les conservateurs n'ont pas été optimisés à leur plus bas niveau d'efficacité.

### Exemple 10 : Formulation Gel crème Vitafruit

| Ingrédient / Nom commercial | Nom INCI | *%* |
|---|---|---|
| Phase A | | |
| Eau purifiée | Water/Aqua | Qs. 100 |
| Tetrasodium EDTA | Tetrasodium EDTA | 0,05 |
| Lubrajel^{™} Oil Free hydrogel^{∗} | Glycerin (and) Glyceryl Acrylate/Acrylic Acid Copolymer (and) PVM/MA Copolymer | 7,00 |
| Optiphen^{™} HD preservative booster | 1,2-Hexanediol | 3,00 |
| Butylene Glycol | Butylene Glycol | 7,00 |
| Glycerin | Glycerin | 5,00 |
| Phase B | | |
| FlexiThix^{™} polymer | PVP | 2,00 |
| Phase C | | |
| Montanov^{™} 68* | Cetearyl Alcohol (and) Cetearyl Glucoside | 0,20 |
| Optiphen 200 preservative | Phenoxyethanol (and) Caprylyl Glycol | 0,90 |
| Ceraphyl^{™} SLK ester | Isodecyl Neopentanoate | 2,50 |
| Lexfeel^{™} N5^{∗} | Diheptyl Succinate (and) Capryloyl Glycerin/Sebacic Copolymer | 1,00 |
| Antaron^{™}/Ganex^{™} Sensory polymer | VP/Acrylates/Lauryl Methacrylate Copolymer | 0,25 |
| Phase D | | |
| Surfin^{™} 96^{∗} | Alcohol | 10,00 |
| Phase E | | |
| RapiThix^{™} A-60 polymer | Sodium Polyacrylate (and) Hydrogenated Polydecene (and) Trideceth-6 | 0,50 |
| Phase F | | |
| Extrait E1 | 1,3-Propanediol (and) Water/Aqua (and) Myrciaria Dubia Fruit Extract | 1,00 |
| Smart 5^{∗} | Isododecane (and) Hydrogenated Tetradecenyl/Methylpentadecene | 5,00 |
| Unicert Yellow 08005-J (Sol. 1*%*) | CI 19140 (yellow 5) | 0,35 |
| Unicert Red 07004-J (sol. 1*%*) | CI 14700 (red 4) | 0,25 |
| Timiron^{™} Silk Gold ^{∗} | CI 77891 (Titanium Dioxide) (and) Mica (and) Tin Oxide | 1,00 |
| PF Caipirinha Fresh G11724962 | Fragrance/Parfum (and) Benzyl Salicylate (and) Citral (and) Limonene (and) Linalool (and) Butylphenyl methylpropional (Lilial) | 0,30 |

Méthode de préparation :
1. Dans le bécher principal ajouter les ingrédients de la phase A, un par un en chauffant à 70°C tout en mélangeant jusqu'à homogénéisation complète.
2. Saupoudrer le polymère FlexiThix^{™} et homogénéiser jusqu'à obtenir un mélange lisse.
3. Dans un bécher à part, chauffer la phase C (sauf Antaron^{™}/Ganex^{™} Sensory polymer) à 70°C jusqu'à homogénéisation. Plonger ensuite l'Antaron/Ganex Sensory polymer dans la Phase C sous agitation.
4. A 70°C ajouter la Phase C (Antaron/Ganex Sensory polymer sont sous forme de poudre mouillée) dans le bécher principal et homogénéiser.
5. Refroidir.
6. A 25°C jouter la Phase D lentement et mélanger jusqu'à homogénéisation complète.
7. Ajouter la Phase E et mélanger jusqu'à homogénéisation complète (la viscosité augmente).
8. A température ambiante, prémélanger la Phase F sous agitation, ajouter le contenu du bécher principal et mélanger bien.
9. Arrêter lorsque le mélange est de couleur uniforme.
10. Apparence : gel crème opalescent orange; pH: 6,0 - 6,5 ; Viscosité (D0): 25000 - 40000 cps (Brookfield RVT/Spindle B/5 RPM/1 minute/25°C).
11. La conservation de la formule a été validée par un test de vieillissement accéléré de 3 mois et un test d'efficacité après 28 jours. Toutefois, les conservateurs n'ont pas été optimisés à leur plus bas niveau d'efficacité.

### Références bibliographiques

(1) Myoda, T.; Fujimura, S.; Park, B.; Nagashima, T.; Nakagawa, J.; Nishizawa, M. Int. J. Food Agric. Environ. 2010, 8 (2), 304.
(2) Yazawa, K.; Suga, K.; Honma, A.; Shirosaki, M.; Koyama, T. J. Nutr. Sci. Vitaminol. (Tokyo) 2011, 57 (1), 104.
(3) Kaneshima, T.; Myoda, T.; Toeda, K.; Fujimori, T.; Nishizawa, M. Biosci. Biotechnol. Biochem. 2017, 81 (8), 1461.
(4) Fracassetti, D.; Costa, C.; Moulay, L.; Tomâs-Barberân, F. A. Food Chem. 2013, 139 (1-4), 578.
(5) Zanatta, C. F.; Mercadante, A. Z. Food Chem. 2007, 101 (4), 1526.
(6) Zanatta, C. F.; Cuevas, E.; Bobbio, F. O.; Winterhalter, P.; Mercadante, A. Z. J. Agric. Food Chem. 2005, 53 (24), 9531.
(7) Akter, M. S.; Oh, S.; Eun, J.-B.; Ahmed, M. Food Res. Int. 2011, 44 (7), 1728.
(8) Justi, K. C.; Visentainer, J. V.; Evelâzio de Souza, N.; Matsushita, M. Arch. Latinoam. Nutr. 2000, 50 (4), 405.
(9) Zapata, S. M.; Dufour, J.-P. J. Sci. Food Agric. 1993, 61 (3), 349.

## Revendications

1. Extrait de pulpe et de peau du fruit séché de *Myrciaria dubia* dépourvu de graines dont la maturité est d'au moins 70 *%* et dont le ratio de matières solides solubles/acidité est compris entre 2,5 et 3, **caractérisé en ce qu'**il est riche en acides organiques et **en ce qu'**il est obtenu à partir de la pulpe et de la peau du fruit séché de *Myrciaria dubia* par extraction solide-liquide dans un solvant hydro-polyalcoolique choisi parmi un solvant hydro-glycérolique et/ou hydro-glycolique, sous agitation classique ou avec ultrasons.

2. Extrait selon la revendication 1, **caractérisé en ce que** le solvant hydro-polyalcoolique est un mélange compris entre 30 et 90 % de glycérol et/ou de glycol dans l'eau.

3. Extrait selon l'une des revendications 1 ou 2, **caractérisé en ce qu'**il comprend par rapport à la matière sèche de l'extrait total entre 13,5 *%* et 80,5 % d'acides organiques dont les acides malique, citrique et ascorbique ; entre 4,0 *%* et 22,0 *%* de sucres totaux dont notamment le glucose, le fructose, le saccharose et le maltose ; entre 0,5 *%* et 3 *%* de minéraux dont potassium, magnésium, calcium, sodium, manganèse, zinc et fer.

4. Extrait selon l'une des revendications 1 à 3, **caractérisé en ce qu'**il comprend une quantité de polyphénols comprise entre 0,08 *%* et 2,00 *%*.

5. Procédé d'obtention d'un extrait selon l'une des revendications 1 à 4, **caractérisé en ce qu'**il comprend les étapes suivantes selon lesquelles :
a) on met en dispersion et on extrait dans un solvant hydro-polyalcoolique la pulpe et la peau du fruit séché de *Myrciaria dubia* dépourvu de graines dont la maturité est d'au moins 70 *%* et dont le ratio de matières solides solubles/acidité est compris entre 2,5 et 3;
b) on sépare la phase liquide chargée en composés d'intérêt de la phase solide par décantation et/ou centrifugation et/ou filtration clarifiante de l'extrait obtenu suite à l'étape a),
c) on effectue une microfiltration de l'extrait liquide obtenu à l'étape b) pour éliminer les particules solides végétales,
d) éventuellement on purifie l'extrait liquide obtenu à l'étape c) par filtration, ultrafiltration et/ou nanofiltration,
e) éventuellement on peut sécher l'extrait de *Myrciaria dubia* liquide obtenu par exemple par atomisation, lyophilisation ou zéodratation, de manière à obtenir un extrait de *Myrciaria dubia* solide avec éventuellement un support de séchage tel que de la maltodextrine.

6. Procédé selon la revendication 5, **caractérisé en ce que** le solvant hydro-polyalcoolique est un mélange comprenant de 10 *%* à 90 *%* en poids de glycérol et/ou de 1,3-propanediol dans de l'eau déminéralisée par rapport au poids total solvant plus eau.

7. Procédé selon la revendication 6, **caractérisé en ce qu'**il est obtenu par extraction solide-liquide, avec un solvant hydro-glycolique composé de 60 *%* de 1,3-propanediol et 40 % d'eau déminéralisée.

8. Procédé selon l'une des revendications 5 à 7, **caractérisé en ce qu'**à l'étape a) la température est comprise entre 4°C et 100°C, la durée d'extraction varie de 10 minutes à 4 heures et l'extraction est effectuée sous agitation classique ou avec ultrasons.

9. Composition cosmétique, **caractérisée en ce qu'**elle comprend, un extrait de pulpe et de peau du fruit de *Myrciaria dubia* selon l'une des revendications 1 à 4, et un milieu physiologiquement acceptable.

10. Composition selon la revendication 9, **caractérisée en ce que** l'extrait de la pulpe et de la peau du fruit de *Myrciaria dubia* est présent dans la composition à une concentration de 0,1 à 3 *%* par rapport au poids total de la composition.

11. Composition selon la revendication 10, **caractérisée en ce que** l'extrait de la pulpe et de la peau du fruit de *Myrciaria dubia* est présent dans la composition à une concentration de 0,1 à 1,5 *%* par rapport au poids total de la composition.

12. Composition selon l'une des revendications 9 à 11, **caractérisée en ce qu'**elle est formulée pour être appliquée topiquement sur la peau.

13. Utilisation cosmétique d'une composition selon l'une des revendications 9 à 12, pour réduire les signes de fatigue de la peau.

14. Utilisation cosmétique d'une composition selon la revendication 13, dans laquelle les signes de fatigue de la peau sont choisis parmi la présence de poches et de cernes sous les yeux, le teint terne, la sécheresse de la peau, l'accentuation des ridules et rides telles que les rides du front ou de la patte d'oie, l'accentuation du relâchement de la peau, un accroissement de la fragilité des ongles et une réduction de la ligne des cheveux.

15. Utilisation cosmétique d'une composition selon l'une des revendications 13 ou 14, pour augmenter la synthèse d'ATP cellulaire, augmenter l'activité de la créatine kinase, augmenter l'expression de collagène de type I, augmenter la synthèse de PFKFB3, diminuer le niveau d'ARN messager de 11b-HSD1 et augmenter le niveau d'ARN messager de 11b-HSD2.

## Patentansprüche

1. Extrakt aus dem Fruchtfleisch und der Schale der getrockneten, entkernten *Myrciaria dubia*-Frucht, dessen Reifegrad mindestens 70 *%* beträgt und dessen Verhältnis von löslichen Feststoffen zu Säure zwischen 2,5 und 3 liegt, **dadurch gekennzeichnet, dass** er reich an organischen Säuren ist und dass er aus dem Fruchtfleisch und der Schale der getrockneten *Myrciaria dubia*-Frucht durch Fest-Flüssig-Extraktion in einem hydro-polyalkoholischen Lösungsmittel, ausgewählt aus einem Hydroglycerinund/oder Hydroglykollösungsmittel, unter herkömmlichem Rühren oder mit Ultraschall bekommen wird.

2. Extrakt nach Anspruch 1, **dadurch gekennzeichnet, dass** das hydropolyalkoholische Lösungsmittel eine Mischung aus 30 bis 90 *%* Glycerin und/oder Glykol in Wasser ist.

3. Extrakt nach Anspruch 1 oder 2, **dadurch gekennzeichnet, dass** er, bezogen auf die Trockenmasse des Gesamtextrakts, zwischen 13,5 *%* und 80,5 *%* organische Säuren, darunter Apfel-, Zitronen- und Ascorbinsäure, Gesamtzucker zwischen 4,0 *%* und 22,0 *%*, darunter insbesondere Glucose, Fructose, Saccharose und Maltose, Mineralstoffe zwischen 0,5 *%* und 3 *%*, darunter Kalium, Magnesium, Calcium, Natrium, Mangan, Zink und Eisen, enthält.

4. Extrakt nach Anspruch 1 bis 3, **dadurch gekennzeichnet, dass** er eine Menge an Polyphenolen zwischen 0,08 *%* und 2,00 *%* umfasst.

5. Verfahren zur Gewinnung eines Extrakts nach einem der Ansprüche 1 bis 4, **dadurch gekennzeichnet, dass** es die folgenden Schritte umfasst, nach denen:
a) das Fruchtfleisch und die Schale der getrockneten, entkernten *Myrciaria dubia*-Frucht deren Reifegrad mindestens 70 *%* beträgt und deren Verhältnis von löslichen Feststoffen zu Säure zwischen 2,5 und 3 liegt, in einem hydropolyalkoholischen Lösungsmittel dispergiert und extrahiert werden;
b) die mit den infrage kommenden Verbindungen beladene flüssige Phase wird von der festen Phase durch Dekantieren und / oder Zentrifugieren und / oder klärende Filtration des nach Schritt a) erhaltenen Extrakts getrennt;
c) um die pflanzlichen Feststoffe zu entfernen, wird der in Schritt b) erhaltene flüssige Extrakt mikrofiltriert;
d) gegebenenfalls wird der in Schritt c) erhaltene flüssige Extrakt durch Filtration, Ultrafiltration und / oder Nanofiltration gereinigt,
e) gegebenenfalls kann der erhaltene flüssige Extrakt der *Myrciaria dubia* z.B. durch Sprühtrocknung, Gefriertrocknung oder Zeolith-Trocknung getrocknet werden, um einen festen *Myrciaria dubia*-Extrakt zu erhalten, gegebenenfalls mit einem Trocknungsmedium wie Maltodextrin.

6. Verfahren nach Anspruch 5, **dadurch gekennzeichnet, dass** das hydropolyalkoholische Lösungsmittel eine Mischung ist, die 10 Gew.-*%* bis 90 Gew.-*%* Glycerin und / oder 1,3-Propandiol in entmineralisiertem Wasser, bezogen auf das Gesamtgewicht von Lösungsmittel plus Wasser, umfasst.

7. Verfahren nach Anspruch 6, **dadurch gekennzeichnet, dass** es durch Fest-Flüssig-Extraktion mit einem hydroglykolischen Lösungsmittel, bestehend aus 60 *%* 1,3-Propandiol und 40 *%* entmineralisiertem Wasser, gewonnen wird.

8. Verfahren nach einem der Ansprüche 5 bis 7, **dadurch gekennzeichnet, dass** in Schritt a) die Temperatur zwischen 4 °C und 100 °C liegt, die Extraktionsdauer zwischen 10 Minuten und 4 Stunden liegt und die Extraktion unter herkömmlichem Rühren oder mit Ultraschall durchgeführt wird.

9. Kosmetische Zusammensetzung, **dadurch gekennzeichnet, dass** sie einen Extrakt aus dem Fruchtfleisch und der Schale der *Myrciaria dubia*-Frucht nach einem der Ansprüche 1 bis 4 und ein physiologisch akzeptables Medium enthält.

10. Zusammensetzung nach Anspruch 9, **dadurch gekennzeichnet, dass** der Extrakt aus dem Fruchtfleisch und der Schale der *Myrciaria dubia*-Frucht in der Zusammensetzung in einer Konzentration von 0,1 bis 3 *%*, bezogen auf das Gesamtgewicht der Zusammensetzung, vorhanden ist.

11. Zusammensetzung nach Anspruch 10, **dadurch gekennzeichnet, dass** der Extrakt aus dem Fruchtfleisch und der Schale der *Myrciaria dubia*-Frucht in der Zusammensetzung in einer Konzentration von 0,1 bis 1,5 *%*, bezogen auf das Gesamtgewicht der Zusammensetzung, vorhanden ist.

12. Zusammensetzung nach einem der Ansprüche 9 bis 11, **dadurch gekennzeichnet, dass** sie so formuliert ist, dass sie topisch auf die Haut aufgetragen werden kann.

13. Kosmetische Verwendung einer Zusammensetzung nach einem der Ansprüche 9 bis 12 zur Verringerung von Ermüdungserscheinungen der Haut.

14. Kosmetische Verwendung einer Zusammensetzung nach Anspruch 13, wobei die Anzeichen von Hautmüdigkeit ausgewählt sind aus dem Vorhandensein von Schwellungen und dunklen Ringen unter den Augen, einem fahlen Teint, trockener Haut, einer Betonung von feinen Linien und Falten wie Stirnfalten oder Krähenfüßen, einer Betonung der Erschlaffung der Haut, einer Zunahme der Brüchigkeit der Nägel und einer Verringerung des Haaransatzes.

15. Kosmetische Verwendung einer Zusammensetzung nach einem der Ansprüche 13 oder 14 zur Erhöhung der Synthese von zellulärem ATP, Erhöhung der Aktivität der Kreatinkinase, Erhöhung der Expression von Kollagen Typ 1, Erhöhung der Synthese von PFKFB3, Verringerung des 11b-HSD1-Messenger-RNA-Spiegels und Erhöhung des 11b-HSD2-Messenger-RNA-Spiegels.

## Claims

1. Extract of pulp and skin of the dried fruit of *Myrciaria dubia* devoid of seeds having a maturity of at least 70*%* and having a soluble solids/acidity ratio of between 2.5 and 3, **characterised in that** it is rich in organic acids and **in that** it is obtained from the pulp and skin of the dried fruit of *Myrciaria dubia* by solid-liquid extraction in a hydro-polyalcoholic solvent selected from a hydro-glycerol and/or a hydro-glycolic solvent, with conventional stirring or with ultrasound.

2. Extract according to claim 1, **characterised in that** the hydro-polyalcoholic solvent is a mixture of between 30 and 90*%* glycerol and/or glycol in water.

3. Extract according to any one of claims 1 or 2, **characterised in that** it comprises relative to the dry matter of the total extract between 13.5*%* and 80.5*%* of organic acids including malic, citric and ascorbic acids; between 4.0*%* and 22.0*%* of total sugars including glucose, fructose, sucrose and maltose; between 0.5*%* and 3*%* of minerals including potassium, magnesium, calcium, sodium, manganese, zinc and iron.

4. Extract according to any one of claims 1 to 3, **characterised in that** it comprises an amount of polyphenols between 0.08*%* and 2.00*%*.

5. Process for obtaining an extract according to any one of claims 1 to 4, **characterised in that** it comprises the following steps according to which:
a) the pulp and skin of the dried fruit of *Myrciaria dubia* devoid of seeds having a maturity of at least 70*%* and having a soluble solids/acidity ratio of between 2.5 and 3 is dispersed and extracted in a hydro-polyalcoholic solvent;
b) the liquid phase loaded with compounds of interest is separated from the solid phase by decantation and/or centrifugation and/or clarifying filtration of the extract obtained following step a),
c) a microfiltration of the liquid extract obtained in step b) is performed to remove the plant solid particles,
d) optionally the liquid extract obtained in step c) is purified by filtration, ultrafiltration and/or nanofiltration,
e) optionally the liquid *Myrciaria dubia* extract obtained can be dried, for example by spraying, lyophilisation or zeodration, so as to obtain a solid *Myrciaria dubia* extract, optionally with a drying substrate such as maltodextrin.

6. Process according to claim 5, **characterised in that** the hydro-poly alcoholic solvent is a mixture comprising from 10*%* to 90*%* by weight of glycerol and/or 1,3-propanediol in demineralised water relative to the total weight of solvent plus water.

7. Process according to claim 6, **characterised in that** it is obtained by solid-liquid extraction with a hydro-glycolic solvent composed of 60*%* of 1,3-propanediol and 40*%* of demineralised water.

8. Process according to any one of claims 5 to 7, **characterised in that** in step a) the temperature is between 4°C and 100°C, the extraction time varies from 10 minutes to 4 hours and the extraction is carried out with conventional stirring or with ultrasound.

9. Cosmetic composition, **characterised in that** it comprises a pulp and skin extract of the fruit of *Myrciaria dubia* according to any one of claims 1 to 4, and a physiologically acceptable medium.

10. Composition according to claim 9, **characterised in that** the extract of the pulp and the skin of the fruit of *Myrciaria dubia* is present in the composition at a concentration of 0.1 to 3*%* relative to the total weight of the composition.

11. Composition according to claim 10, **characterised in that** the extract of the pulp and the skin of the fruit of *Myrciaria dubia* is present in the composition at a concentration of 0.1 to 1.5*%* relative to the total weight of the composition.

12. Composition according to any one of claims 9 to 11, **characterised in that** it is formulated to be applied topically to the skin.

13. Cosmetic use of a composition according to any one of claims 9 to 12, to reduce the signs of fatigue of the skin.

14. Cosmetic use of a composition according to claim 13, in which the signs of fatigue of the skin are selected from the presence of bags and dark circles under the eyes, dull complexion, dryness of the skin, accentuation of fine lines and wrinkles such as wrinkles on the forehead or crow's-feet, accentuation of looseness of the skin, an increase in the fragility of nails and a reduction of hair line.

15. Cosmetic use of a composition according to any one of claims 13 or 14, to increase the synthesis of cellular ATP, to increase the activity of creatine kinase, to increase the expression of type I collagen, to increase the synthesis of PFKFB3, to decrease the level of messenger RNA by 11b-HSD1 and to increase the level of messenger RNA by 11b-HSD2.
